# EUROPEAN PATENT SPECIFICATION

(11) **EP 4 486 718 B1**
(45) Date of publication and mention of the grant of the patent: **08.04.2026**
(21) Application number: 23706634.5
(22) Date of filing: 27.02.2023
(51) Int. Cl.: C07C 51/43, C07C 59/08

(54) **METHOD FOR MANUFACTURING SODIUM LACTATE SOLID PARTICLES AND USE THEREOF**
VERFAHREN ZUR HERSTELLUNG VON FESTSTOFFPARTIKELN AUS NATRIUMLACTAT UND DEREN VERWENDUNG
PROCÉDÉ DE FABRICATION DE PARTICULES SOLIDES DE LACTATE DE SODIUM ET LEUR UTILISATION

(30) Priority: 28.02.2022 EP 22159213
(43) Date of publication of application: 08.01.2025
(73) Proprietor: Purac Biochem B.V., 4206 AC Gorinchem (NL)
(72) Inventor: JANSEN, Peter Paul, 4206 AC Gorinchem (NL); VAN KRIEKEN, Jan, 4206 AC Gorinchem (NL); VAN STRIEN, Rick Jeffrey, 4206 AC Gorinchem (NL)
(74) Representative: De Vries & Metman
(86) International application number: PCT/EP2023/054866
(87) International publication number: WO 2023/161483

(56) References cited:
- WO-A1-03/031385
- CN-A- 110 642 702
- NL-A- 7 106 959

## Description

The invention relates to a method for manufacturing sodium lactate (hereafter also referred to as NaL) in a solid form, in particular in a crystalline form.

Sodium lactate (NaL) is used in a broad spectrum of applications, such as pharmaceutical, food and personal care applications. Depending on the application, the NaL is used in aqueous or solid form. A known form of solid NaL is a powder, which allows the use of solid powder processing and dosing techniques. NL7106959 describes a method to produce sodium lactate powder using spray drying.

WO 2019/068798 describes a number of problems of NaL in powder form. In particular NaL is hygroscopic, and it may become sticky over time in storage or during processing. The stickiness causes the formation of lumps or cakes in the powder material and has a negative impact on the flow properties and processability.

An example of a known process for producing NaL powder with an improved stability is disclosed in WO 03/031385 A1, filed in the name of the current applicant. The document describes the production of alkali metal lactate in powder form, wherein a concentrate solution of alkali metal lactate is processed with a carrier material, for example starch or silicate, in a cooled mixer/extruder to form a solid alkali metal lactate powder, optionally followed by processing in a conical flourmill or a hammer mill to further decrease the particle size. The NaL powder prepared making this method was shown to be relatively stable when exposed to a humid atmosphere, allowing a longer time for processing before the material becomes too sticky.

Production of powder using an extrusion method may result in high shear and stress on the product and/or on the extruder itself. The shear forces in the extruder have the risk of causing undesired impurities from wear of the extruder contact surface, which is typically a metal or ceramic material. This risk may require additional product quality control and/or additional purification steps to remove the impurities. Removal of the impurities is of particular importance in applications such as pharmaceutical and food applications.

WO 2019/068798 describes lactate salt particles with a particle size in the range of 120 to 1,200 µm (measured using laser diffraction), that are provided with a coating layer in order to improve the processability of the lactate salt powder. However, applying the coating introduces complex additional process steps, and the coating material introduces new additives to the lactate product that are undesirable or unacceptable for certain applications.

CN 110642702 A describes an industrialized preparation device for high-purity, low-water-content sodium lactate powder and a corresponding preparation method. In the process of this reference a screw feeder is used.

It is an objective of the invention to alleviate or even obviate the aforementioned disadvantages of the prior art. More in particular, it is an object of the invention to provide a method for manufacturing NaL in a solid form with an improved processability compared to sodium lactate powder. It is another object of the invention to provide a method to produce solid NaL that reduces the risk of impurities in the product compared to extrusion methods.

The invention provides a method for manufacturing sodium lactate (NaL) solid particles, characterized in that the method comprises the following steps: providing a melt of NaL, wherein the melt has a chemical purity of at least 95 %.wt., and a water content of less than 5% wt., applying at least a part of said melt on a cooling surface, which surface is provided with at least partially crystalline seed particles of NaL, crystallizing the applied NaL on the cooling surface forming solid NaL, and collecting at least part of the crystallized NaL from the cooling surface as solid NaL particles.

The solid NaL particles formed using this method were found to have a better processability than the corresponding NaL powder prepared from the same NaL melt, without the need for further additives such as anti-caking agents. Specific advantages of the aspects of the invention, as well as of specific embodiments thereof, will become apparent from the further specification.

The provided NaL melt preferably has a high NaL purity and low water content. For the crystallization, the cooling surface is kept at a temperature below the melting temperature of the NaL. The melting temperature of suitable NaL is typically in the range of 155-162 °C, varying with water content and impurities. A larger temperature gap between melting temperature and the temperature of the cooling plate results in faster solidification.

The crystallized NaL may contain a minor fraction of amorphous NaL. However, if the solification is too fast, it was found the resulting product properties become less favorable, possibly due to the presence of too much amorphous NaL.

Preferably, the NaL melt has a chemical purity of at least 95 %. wt., preferably at least 99 %. wt. and more preferably 99.5 %.wt.

It is preferred the melt has a water content of less than 5%, preferably less than 1% wt., more preferably less than 0.5% wt. High water content in the NaL melt increases the risk for obtaining particles with instability and/or tackiness.

The cooling surface could be a flat surface or curved and is preferably moving relative to an applicator of the melt of NaL. Examples of cooling surfaces included the surface of a cooled disc or a cooled belt conveyor, or the inner or outer surface of a cooled rotating drum.

The at least partially crystalline seed particles may be deposited on the surface prior to applying the melt of NaL, but they may also be at least partially crystalline NaL material remaining on the surface after collecting NaL particles from the same surface, for instance in an earlier batch run of the process, or during the process when the process is repeated on the same cooling surface in a continuous or semi-continuous way.

The solid NaL can be collected from the cooling surface, preferably in a mechanical way such as scraping or cutting.

In a preferred embodiment, the melt of NaL is applied as droplets on the cooling surface. The droplets solidify to particles. Droplets can be applied by a nozzle. The size of the droplets can be controlled by the size and shape of the nozzle, and dosing equipment such as a plunger or piston.

Preferably the distance between separate droplets on the surface is at least 5% of the average diameter, more preferably at least 10%. Applying the droplets at separate positions at the surface prevents fusing of particles during the solidifying step.

In a preferred embodiment, the melt of NaL is applied in shaped receptacles positioned in the cooling surface. Receptacles allow for better control of the position and shape of the applied molten NaL.

The melt of can be applied at in a predetermined pattern. Patterns can be used for optimal use of the available cooling surface area. Common patterns for droplets would for instance be various known grid patterns.

In a preferred embodiment, the solidified NaL is collected as pastilles. Pastille shapes typically have at least a flat surface and a round surface, for instance a disc shape or a hemi-spheroid shape, or shapes derived thereof. Such shapes can be conveniently formed by the controlled application of a drop molten liquid on a flat cooling surface. Pastilles were found to be a convenient form to dose and process NaL in a plethora of applications, such as the manufacture of a pharmaceutical product, a food product, a cleaning product or a personal care product.

It is preferred if the solidified NaL is at least partially crystalline. This implies the solidified NaL contains more crystalline material than the crystalline fraction already present in the seed particles. Methods to determine the percentage crystallinity versus amorphous material include X-ray diffraction, density measurements or differential scanning calorimetry (DSC). Visual inspection will also show whether a material is mostly crystalline or amorphous.

In a preferred embodiment, the solidified NaL is at least 50% crystalline, preferably at least 70% crystalline, more preferably at least 90% crystalline, as measured by DCS.

In a preferred embodiment, the temperature of the cooling surface during the solidification step ranges between 35 and 160 °C, preferably between 100 and 155 °C. In this temperature range, at least partially crystalline particles are formed in a relatively short time. Whereas the needed crystallization time depends on the abilities such as cooling capacity of equipment used, preferably the solidification time during the solidification step is under 10 minutes, preferably under 5 minutes, more preferably under 2 minutes. A higher temperature of the cooling surface makes removal from the cooling surface easier, possibly due to an increased quality of crystallization.

The preferred difference between the melting temperature of the NaL and the temperature of the cooling surface during solidification is kept between 5-50 °C, more preferably between 10-30 °C. In this ranged, a relatively high crystallinity is achieved at a relatively fast crystallization speed.

It is preferred that at least during the solidifying step the cooling surface is kept in an atmosphere having a relative humidity (RH) of less than 30%, preferably less than 20 %. Surprisingly, it was found that the formed particles show very good stability and processability compared to the same particles prepared at higher humidity.

In a preferred embodiment, in the step of removing the solidified NaL from the cooling surface, residual crystalline particles remain on said surface as seed particles for crystallization of a subsequent application of melt on the same cooling surface. This allows for a batch-continuous or full continuous process. This could be done by recycling the cooling surface, for instance re-using a cooling plate after particles are collected. This is particularly attractive when using a continuously moving cooling surface, such as an endless conveyor belt, rotating disc or rotating drum. In some embodiments crystallization rate increased by the presence of crystalline seed crystals on the cooling surface.

In a preferred embodiment, the used NaL is at least 90 %. wt., preferably at least 95 %. wt., more preferably 98 %.wt. enantiomerically pure. Although for racemic mixtures the method also improved processability of the resulting particles compared to powder, enantiomeric pure material gave better stability and flowability properties under the same process conditions.

It is preferred if the cooling surface is incorporated in a melt cooling device, such as a cooling belt device, a pastillator device or a drum flaker device. Such equipment is commercially available and can be made suitable for the process described herein.

Preferably, the melt cooling device further comprises a scraper for at least substantially removing the crystallized NaL from the cooling surface such as a scraper, preferably made of a metal-free material such as a plastic or a ceramic material. Preferably, the material is removed using low mechanical friction between the scraper and the cooling surface. The scraper position with respect to the cooling surface can be adjusted to not completely remove all of the NaL, allowing to re-use remaining crystalline NaL as seed crystals for subsequent particles to be manufactured on the same surface.

The invention further provides particles of NaL, wherein the NaL particles are at least partially crystalline, with a chemical purity of at least 95 %. wt., and a water content of less than 5%, and an average weight from 1-100 mg per particle. These particles show a better processability than powdered NaL without the need for additives such as anti-caking agents. The tackiness of the particles remains low for at least 1 hour typically from 20 ° and an air humidity of 30%. These particles are substantially larger than powder particles, but still allow for precise dosing. The particles may be obtainable or obtained by the method described herein.

Preferably, the particles have an average weight of from 10-80 mg per particle, preferably from 20-60 mg per particle. Such particles showed a good combination of flowability and stability when exposed to air humidity.

It is preferred if the particles have an average weight distribution with a standard deviation of at most 40% of the average weight, preferably at most 20% of the average weight, and more preferably at most 10% of the average weight.

In a preferred embodiment, the particles have a largest dimension between 0.5 and 10.0 mm, preferably between 1.0 and 8.0 and more preferably between 2.0 and 7.0 mm. Such particles showed a good combination of flowability and stability when exposed to air humidity.

It is preferred if the particles have a disc shape or a hemi-spheroid shape. Such particles showed a good combination of flowability and stability when exposed to air humidity. The form of a particle may deviate from perfect discs or hemi-spheroids and may for instance have an ellipsoid base shape. The particles are particularly advantageous when also having the average weight and dimensions as mentioned herein.

Preferably, the particles are non-tacky. Non-tackiness implies that, when exposed to a processing temperature and humidity, typically from 20 ° and an air humidity of 30%, particles do not stick together at least for 1 hour. Due to the non-tackiness, the material is essentially free flowing and easy to process. The lack of tackiness for at least 1 hour typically from 20 ° and an air humidity of 30% makes the particles essentially free flowing. Such particles have excellent processability, even after extended storage life.

It is preferred if the particles are essentially free of additives, in particular essential free of anti-caking agents. The absence of additives makes the particles particularly attractive for use in applications requiring a high purity, such as the preparation of pharmaceutical formulations. Essentially free means less than 0.5 wt.% additives, preferably less than 0.1 wt.%. Examples of commonly used anti-caking additives include tricalcium phosphate, powdered cellulose, magnesium stearate, sodium bicarbonate, calcium phosphate, sodium silicate, silicon dioxide, calcium silicate, magnesium trisilicate, talcum powder, stearic acid, and starch powder.

The invention further provides the use of solid sodium lactate (NaL) particles according to the invention, in the preparation of a pharmaceutical product, a food product, a cleaning product or a personal care product. The improved processability of the particles without the need for additives, makes the dosing of the particles in the preparation process more convenient compared to NaL powder.

The invention is further elucidated by the following experiments and figures, which are to be construed as illustrative only and not as being limitative.

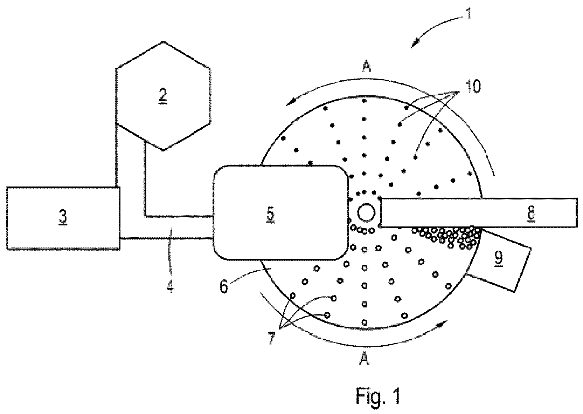

Figure 1 shows a schematic view of a set-up for carrying out a method according to the invention.

### Example 1: Melt preparation

Sodium L-lactate 60% in aqueous solution, pharmaceutical grade (1.6 kg) was concentrated by means of a rotary evaporator at 7 cmHg. Bath temperature was maintained for 2 hours at 100°C, followed by 1 hour at 150°C. The resulting product was a viscous melt at 150°C, with a water content of 0.7 %. wt.

### Example 2: Comparative crystallization examples

Approximately 100 grams of solid sodium L-lactate powder (> 99 percent chemical purity, free from anti-caking agent), was covered with a glass and heated in an oven at 180 °C under a nitrogen blanket in order to obtain a melt. A cooling plate was provided which was kept at a temperature as specified in Table 1 using a thermostatic bath. An amount of the melt was provided on the cooling plate. For the Examples in which a seed crystal was provided on the cooling plate, an amount of crystalline NaL powder was spread evenly onto the cooling plate surface before application of the melt, and the melt was applied on top of the powder.

Examples 1-5 were performed at different temperatures without the application of seed crystals, whereas Examples 6-10 used the same temperature series but with the application of seed crystals. The resulting solidification of material (if any) was timed visually, and inspected under a microscope.

**Table 1 - Experimental set-up and results**

| | Cooling plate temperature [°C] | Seed crystals on cooling plate | Solidification/Crystallization |
|---|---|---|---|
| Example 1 | 25 | No | No visible solidification within 5 min |
| Example 2 | 35 | No | No visible solidification within 5 min |
| Example 3 | 75 | No | No visible solidification within 5 min |
| Example 4 | 100 | No | No visible solidification within 5 min |
| Example 5 | 125 | No | No visible solidification within 5 min |
| Example 6 | 25 | Yes | Seed crystal trapped in amorphous phase |
| Example 7 | 35 | Yes | Seed crystal trapped in amorphous phase |
| Example 8 | 75 | Yes | Crystallization (> 1 min required) |
| Example 9 | 100 | Yes | Crystallization (approximately 1 min) |
| Example 10 | 125 | Yes | Crystallization (< 1 min) |

The results indicate that the rate of crystallization increases by the presence of crystalline seed crystals on the cooling plate AND the temperature of the cooling plate. This also indicates that when the temperature difference between the melting temperature and cooling plate temperature is too large, mostly amorphous NaL is produced, resulting in less favorable processability of the resulting particles. Furthermore, a cooling plate temperature of at least 100 °C increases the rate of crystallization.

### Example 3: NaL Pastille particle preparation

A setup 1 comprising an ANDRITZ Gouda disc pastillator was provided, which is schematically shown in Figure 1, comprising a melt vessel 2 from which a melt is transportable by plungers 3 and through a heated piping 4 towards a nozzle head 5 with a number of nozzles, for depositing the melt on a circular cooling plate 6 as individual non-touching droplets 7 with an essentially hemi-spheroid shape.

The cooling plate 6 is rotatable in direction A, providing time for the droplets to crystallize. The cooling plate 6 is furthermore provided with a PTFE (teflon) knife 8, downstream of the nozzle head 5, for removing the particles from the cooling plate 6, and a collection tray 9, arranged downstream of the knife 8, for collecting the particles removed by the knife 8. The knife action leaving particles on the surface which can act as seed crystals 10 for repeating the process with a further deposition of NaL melt.

The pastillator was insulated and rinsed with acetone for removing moist and contaminations and optimizing heat transfer and arranged in a hood provided with a continuous dry air supply and suction point for expelling any excess moist, in order to keep the relative humidity below 20%. The oil temperature of the pastillator was set to 190 °C, forcing any excess moist out of the equipment. The rotatable cooling plate of the disc pastillator was kept under continuous nitrogen supply for keeping the cooling plate dry and inert.

5 kilograms of solid sodium L-lactate powder (>99 percent chemical purity, free from anti-caking agent, melting temperature around 160 °C, water content 1 % wt.) was charged into the melting vessel under continuous dry nitrogen flow of the pastillator, and heated and homogenized under these conditions at 190 °C for 3 hours, with occasional manual stirring, after which the entire batch had molten into a homogeneous and transparent melt.

Crystalline NaL powder was applied onto the pastillator cooling plate to act as seed crystals. The cooling plate had a smooth flat stainless-steel surface.

Dosed amounts of NaL melt were applied as non-touching droplets onto the surface provided with seed crystals through a controlled nozzle. The diameter of the applied droplets was adjusted by adjustment of the dosing amount of NaL per droplet by setting the automated plungers of the nozzle of the pastillator. The dosing amount was set to 40 mg NaL per droplet for all tests. The nozzle dosing was set to apply the same amount of NaL per droplet. The droplets were applied in a regular grid pattern with sufficient space between the droplet positions, ensuring that the droplets do not touch neighboring droplets. Some minor flowing of the molten NaL may still occur after application on the cooling surface, showing some slightly wider diameters for pastilles produced at higher cooling plate temperatures.

Solidified particles were removed from the plate with a PTFE (Teflon) scraper and collected in aluminum cans sealed in PE bags with an outer aluminum lining under nitrogen supply. The collected particles had a pastille shape, essentially a hemi-spheroid shape with a round flat base in contact with the cooling surface, and a curved spheroid surface forming the top side of the pastille. The average weight of each pastille was around 40 mg ±1 mg.

The Teflon scraper had low mechanical friction with the stainless-steel surface. During and after application of the melt, the rotational speed of the cooling plate was set to obtain a residence time of 99 seconds between application of the melt onto and removal of the formed pastille from the cooling plate. DSC measurements showed that the collected particles were mostly crystalline.

It was found that some minor amounts of crystalline NaL particles remained on the cooling surface after scraping off the pastilles. The remaining NaL were found to be sufficient to be used as seed particles for repeating the process with another batch of molten NaL deposited on the same cooling surface, without the need of adding fresh seed particles.

In each of the Examples of this experiment, the rate of crystallization and the appearance of the particles were compared to a baseline setting (Example 11). After removal of the pastilles, some residual material was present on the cooling surface, which acted as seed crystals, without addition of further seed crystals. The results are shown in Table 2.

**Table 2 - Experimental set-up and results**

| | Cooling plate temperature [°C] | Settings | Average particle diameter [mm] | Appearance |
|---|---|---|---|---|
| Example 11 | 125 | Baseline | 3.5 | Fast crystallization to non-tacky and free flowing particles with shiny top; easy removal |
| Example 12 | 125 | Increased feed flow | 6.5 | Similar to Example 11 |
| Example 13 | 140 | Increased cooling plate temperature | 6.5 | Particles more white and less shiny than Example 11, otherwise similar |
| Example 14 | 100 | Decreased cooling plate temperature | 6.5 | Slower crystallization than Example 11, hard to remove from cooling plate |

Crystallized particles were obtained in each of the examples of this experiment. In Example 14, amorphous parts were still observable, however the pastilles obtained in each of the examples did not have a tacky character. An increase in cooling surface temperature makes the removal from the cooling plate easier, suggesting an increased rate of crystallization, as the pastilles are generally also whiter and less shiny. At a temperature of 100 °C, the pastilles were hard to remove from the cooling plate within the set residence time, suggesting incomplete crystallization. The average pastille diameter was determined by manually measuring multiple pastilles in a batch.

In the tested range, adjustment of the nozzle for obtaining particles with an increased size did not show a notable influence on the properties obtained, apart from a bigger average diameter (increasing from approximately 3.5 mm to approximately 6.5 mm) at an approximately same height (with a range of 1.5 to 1.8 mm).

### Example 4: Stability tests

Powdered sodium lactate, prepared as described using a cooled extruder in WO 03/031385, without adding a carrier material, was compared with pastilles prepared according to examples 6-9, made from the same sodium lactate starting material.

5 gram samples of the powder and the pastilles were placed in plastic dishes (4 x 3 cm) and left for 12 hours at 20°C in an atmosphere with humidity kept between 30-40%. For comparison, the same powder and pastilles where also stored in airtight plastic containers.

After 2 hours, the powder started to show stickiness, which was showed by a diminished flowability of the powder when shaking the plastic dish horizontally. The pastilles showed no decrease in flowability after 2 hours.

After 12 hours of storage, the sodium lactate powder had transformed into a liquid solution without solids. The pastilles had maintained their solid form, although a thin liquid film was visible on the surface of the pastilles, and the pastilles showed slight stickiness to other particles and the dish when shaking horizontally.

Both the powder and pastilles kept in the airtight containers had kept their solid forms without showing liquid forming nor stickiness after 12 hours. After opening the containers, the powder, started to become sticky within one hour, whereas the pastilles retained their good processability.

## Claims

1. Method for manufacturing sodium lactate (NaL) solid particles, **characterized in that** the method comprises the following steps:
- providing a melt of NaL wherein the melt has a chemical purity of at least 95 %. wt., and a water content of less than 5% wt.;
- providing a cooling surface, which surface is provided with at least partially crystalline seed particles of NaL;
- applying at least a part of said melt on a cooling surface;
- crystallizing the applied NaL on the cooling surface forming solid NaL, and;
- collecting at least part of the solid NaL from the cooling surface as solid NaL particles.

2. Method according to claim 1, **characterized in that** the melt of NaL is applied as droplets on the cooling surface.

3. Method according to claim 1 or 2, wherein the melt of NaL is applied in shaped receptacles positioned in the cooling surface.

4. Method according to any of the preceding claims, wherein the melt of NaL is applied at in a predetermined pattern for optimal use of the surface area.

5. Method according to any of the preceding claims, wherein the solidified NaL is collected as pastilles.

6. Method according to any of the preceding claims, **characterized in that** the temperature of the cooling surface at least during the crystallizing step ranges between 35 and 160 °C, preferably between 100 and 155 °C.

7. Method according to any of the preceding claims, **characterized in that** at least during the crystallizing step the cooling surface is kept in an atmosphere having a relative humidity (RH)of less than 30%, preferably less than 20 %.

8. Method according to any of the preceding claims, **characterized in that** the melt of NaL is at least 90 %.wt., preferably at least 95 %.wt., more preferably 98 %.wt. enantiomerically pure.

9. Method according to any of the preceding claims, **characterized in that** in the step of collecting the crystallized NaL from the cooling surface, residual crystalline particles remain on said surface as seed particles for crystallization of a subsequent application of melt on the same cooling surface.

10. Method according to any of the preceding claims, **characterized in that** the cooling surface is incorporated in a melt cooling device, such as a cooling belt device, a pastillator device or a drum flaker device.

11. Particles of NaL wherein the NaL particles are at least partially crystalline, with a chemical purity of at least 95 %.wt., and a water content of less than 5%, and an average weight from 1-100 mg per particle.

12. Particles according to claim 11, wherein the particles have an average weight of from 10-80 mg per particle, preferably from 20-60 mg per particle.

13. Particles according to any of the claims 11-12, wherein the particles have a largest dimension between 0.5 and 10.0 mm, preferably between 1.0 and 8.0 and more preferably between 2.0 and 7.0 mm.

14. Particles according to any of the claims 11-13, wherein said particles have a disc shape or a hemi-spheroid shape.

15. Particles according to any of the preceding claims 11-14, wherein the particles are non-tacky.

16. Particles according to any of the preceding claims 11-15, wherein the particles are essentially free of additives, in particular essential free of anti-caking agents.

17. Use of solid sodium lactate (NaL) particles according to any of the preceding claims 11-16, or obtainable by a method according to any of the claims 1-10, in the preparation of a pharmaceutical product, a food product, a cleaning product or a personal care product.

## Patentansprüche

1. Verfahren zur Herstellung von festen Natriumlactat (NaL)-Partikeln, **dadurch gekennzeichnet, dass** das Verfahren die folgenden Schritte umfasst:
- Bereitstellen einer NaL-Schmelze, wobei die Schmelze eine chemische Reinheit von mindestens 95 Gew.-% und einen Wassergehalt von weniger als 5 Gew.-% aufweist;
- Bereitstellen einer Kühlfläche, wobei die Fläche mit zumindest teilweise kristallinen Keimteilchen aus NaL versehen ist;
- Aufbringen mindestens eines Teils der Schmelze auf eine Kühlfläche;
- Kristallisieren des aufgebrachten NaL auf der Kühlfläche, um festes NaL zu bilden, und
- Gewinnen von mindestens einem Teil des festen NaL von der Kühlfläche als feste NaL-Partikel.

2. Verfahren gemäß Anspruch 1, **dadurch gekennzeichnet, dass** die NaL-Schmelze als Tröpfchen auf die Kühlfläche aufgebracht wird.

3. Verfahren gemäß Anspruch 1 oder 2, wobei die NaL-Schmelze in geformte Behälter, welche auf der Kühlfläche angeordnet sind, aufgebracht wird.

4. Verfahren gemäß einem der vorhergehenden Ansprüche, wobei die NaL-Schmelze in einem vorbestimmten Muster für eine optimale Nutzung des Oberflächenbereichs aufgebracht wird.

5. Verfahren gemäß einem der vorhergehenden Ansprüche, wobei das verfestigte NaL als Pastillen gewonnen wird.

6. Verfahren gemäß einem der vorhergehenden Ansprüche, **dadurch gekennzeichnet, dass** die Temperatur der Kühlfläche zumindest während des Kristallisationsschrittes im Bereich zwischen 35 und 160 °C, vorzugsweise zwischen 100 und 155 °C liegt.

7. Verfahren gemäß einem der vorhergehenden Ansprüche, **dadurch gekennzeichnet, dass** zumindest während des Kristallisationsschritts die Kühlfläche in einer Atmosphäre mit einer relativen Feuchtigkeit (RH) von weniger als 30%, vorzugsweise weniger als 20 %, gehalten wird.

8. Verfahren gemäß einem der vorhergehenden Ansprüche, **dadurch gekennzeichnet, dass** die NaL-Schmelze mindestens 90 Gew.-%, vorzugsweise mindestens 95 Gew.-%, stärker bevorzugt 98 Gew.-%, enantiomerenrein ist.

9. Verfahren gemäß einem der vorhergehenden Ansprüche, **dadurch gekennzeichnet, dass**, in dem Schritt des Gewinnens des kristallinen NaL von der Kühlfläche, kristalline Restteilchen auf dieser Fläche als Keimteilchen zur Kristallisation einer nachfolgend aufgebrachten Schmelze auf derselben Kühlfläche verbleiben.

10. Verfahren gemäß einem der vorhergehenden Ansprüche, **dadurch gekennzeichnet, dass** die Kühlfläche in eine Schmelzkühlvorrichtung eingebracht ist, wie einer Kühlbandvorrichtung, einer Pastilliervorrichtung oder einer Flockierwalze.

11. NaL-Partikel, wobei die NaL-Partikel zumindest teilweise kristallin sind, mit einer chemischen Reinheit von mindestens 95 Gew.-% und einem Wassergehalt von weniger als 5 % und einem mittleren Gewicht von 1-100 mg pro Partikel.

12. Partikel gemäß Anspruch 11, wobei die Partikel ein mittleres Gewicht von 10-80 mg pro Partikel , vorzugsweise 20-60 mg pro Partikel aufweisen.

13. Partikel gemäß einem der Ansprüche 11 bis 12, wobei die Partikel eine größte Abmessung zwischen 0,5 und 10,0 mm, vorzugsweise zwischen 1,0 und 8,0 und stärker bevorzugt zwischen 2,0 und 7,0 mm aufweisen.

14. Partikel gemäß einem der Ansprüche 11 bis 13, wobei die Partikel eine Scheibenform aufweisen oder halbkugelförmig ausgebildet sind.

15. Partikel gemäß einem der vorhergehenden Ansprüche 11 bis 14, wobei die Partikel nichtklebend sind.

16. Partikel gemäß einem der vorhergehenden Ansprüche 11 bis 15, wobei die Partikel im Wesentlichen frei von Zusatzstoffen, insbesondere im Wesentlichen frei von Trennmitteln sind.

17. Verwendung von festen Natriumlactat (NaL)-Partikeln gemäß einem der vorhergehenden Ansprüche 11 bis 16, oder erhältlich durch ein Verfahren gemäß einem der Ansprüche 1 bis 10, bei der Herstellung eines pharmazeutischen Produkts, eines Nahrungsmittelprodukts, eines Reinigungsmittels oder eines Körperpflegeprodukts.

## Revendications

1. Procédé de fabrication de particules solides de lactate de sodium (NaL), **caractérisé en ce que** le procédé comprend les étapes suivantes :
- la fourniture d'une masse fondue de NaL, dans lequel la masse fondue présente une pureté chimique d'au moins 95 % en poids et une teneur en eau inférieure à 5 % en poids ;
- la fourniture d'une surface de refroidissement, laquelle surface est pourvue de particules germes de NaL au moins partiellement cristallines ;
- l'application d'au moins une partie de ladite masse fondue sur une surface de refroidissement ;
- la cristallisation du NaL appliqué sur la surface de refroidissement pour former du NaL solide, et ;
- la collecte d'au moins une partie du NaL solide à partir de la surface de refroidissement sous forme de particules solides de NaL.

2. Procédé selon la revendication 1, **caractérisé en ce que** la masse fondue de NaL est appliquée sous forme de gouttelettes sur la surface de refroidissement.

3. Procédé selon la revendication 1 ou 2, dans lequel la masse fondue de NaL est appliquée dans des réceptacles mis en forme positionnés dans la surface de refroidissement.

4. Procédé selon quelconque des revendications précédentes, dans lequel la masse fondue de NaL est appliquée selon un motif prédéterminé pour une utilisation optimale de la surface.

5. Procédé selon quelconque des revendications précédentes, dans lequel le NaL solidifié est collecté sous forme de pastilles.

6. Procédé selon quelconque des revendications précédentes, **caractérisé en ce que** la température de la surface de refroidissement, au moins pendant l'étape de cristallisation, se situe entre 35 et 160 °C, de préférence entre 100 et 155 °C.

7. Procédé selon quelconque des revendications précédentes, **caractérisé en ce que**, au moins pendant l'étape de cristallisation, la surface de refroidissement est maintenue dans une atmosphère présentant une humidité relative (HR) inférieure à 30 %, de préférence inférieure à 20 %.

8. Procédé selon quelconque des revendications précédentes, **caractérisé en ce que** la masse fondue de NaL présente une pureté énantiomérique d'au moins 90 % en poids, de préférence d'au moins 95 % en poids, et plus préférentiellement d'au moins 98 %.

9. Procédé selon quelconque des revendications précédentes, **caractérisé en ce que**, lors de l'étape de collecte du NaL cristallisé à partir de la surface de refroidissement, des particules cristallines résiduelles restent sur ladite surface en tant que particules germes pour la cristallisation d'une application ultérieure de masse fondue sur la même surface de refroidissement.

10. Procédé selon quelconque des revendications précédentes, **caractérisé en ce que** la surface de refroidissement est incorporée dans un dispositif de refroidissement de masse fondue, tel qu'un dispositif à bande de refroidissement, un dispositif de pastillation ou un dispositif de floconnage à tambour.

11. Particules de NaL, dans lesquelles les particules de NaL sont au moins partiellement cristallines, avec une pureté chimique d'au moins 95 % en poids, et une teneur en eau inférieure à 5 %, et un poids moyen de 1 à 100 mg par particule.

12. Particules selon la revendication 11, dans lesquelles les particules présentent un poids moyen de 10 à 80 mg par particule, de préférence de 20 à 60 mg par particule.

13. Particules selon quelconque des revendications 11 à 12, dans lesquelles les particules ont une plus grande dimension entre 0,5 et 10,0 mm, de préférence entre 1,0 et 8,0 mm et plus préférentiellement entre 2,0 et 7,0 mm.

14. Particules selon quelconque des revendications 11 à 13, dans lesquelles lesdites particules ont une forme de disque ou une forme hémisphéroïde.

15. Particules selon quelconque des revendications 11 à 14 précédentes, dans lesquelles les particules ne sont pas collantes.

16. Particules selon quelconque des revendications 11 à 15 précédentes, dans lesquelles les particules sont essentiellement exemptes d'additifs, en particulier essentiellement exemptes d'agents antiagglomérants.

17. Utilisation de particules solides de lactate de sodium (NaL) selon quelconque des revendications 11 à 16 précédentes, ou pouvant être obtenues par un procédé selon quelconque des revendications 1 à 10, dans la préparation d'un produit pharmaceutique, d'un produit alimentaire, d'un produit nettoyant ou d'un produit de soins personnels.
